# FASCICULE DE BREVET EUROPEEN

(11) **EP 2 647 406 B1**
(45) Date de publication et mention de la délivrance du brevet: **29.10.2014**
(21) Numéro de dépôt: 12192673.7
(22) Date de dépôt: 14.11.2012
(51) Int. Cl.: A61N 1/375, H01R 13/40, H01R 13/629, H01R 24/58, H01R 13/58, H01R 43/26

(54) **Fiche de connexion électrique pour sonde multipolaire de dispositif médical implantable actif**
Elektrischer Anschlussstift für multipolare Sonde einer aktiven implantierbaren medizinischen Vorrichtung
Electrical connection plug for multipolar probe of an active implantable medical device

(30) Priorité: 04.04.2012 FR 1253103
(43) Date de publication de la demande: 09.10.2013
(73) Titulaire: Sorin CRM SAS, 92140 Clamart (FR)
(72) Inventeur: Viatgé, Hélène, 92120 Montrouge (FR); Roussin, Mylène, 10035 Mazze (TO) (IT); Di Maio, Luciano, 10122 Torino (TO) (IT)
(74) Mandataire: Dupuis-Latour, Dominique

(56) Documents cités:
- US-A1- 2010 211 144
- US-A1- 2011 144 722
- US-B1- 6 248 080
- US-B1- 7 241 180

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle ...

Il existe des systèmes de connexion normalisés permettant de garantir l'interchangeabilité des sondes et des générateurs produits par différents fabricants.

Ainsi, les standards dits "IS-1" et "IS-4" définissent un certain nombre de caractéristiques dimensionnelles et électriques relatives aux sondes de délivrance d'impulsions de stimulation (ou de resynchronisation). Pour les sondes de défibrillation, où les contraintes électriques sont plus sévères compte tenu de l'énergie élevée devant transiter du générateur à la sonde, les standards dits "DF-1" et "DF-4" définissent les caractéristiques dimensionnelles et électriques du système de connexion.

L'invention concerne plus particulièrement une fiche de connexion électrique multipôle pour une telle sonde, par exemple une sonde monocorps pourvue à la fois d'électrodes de stimulation et de choc.

La complexité de ces sondes, qui intègrent déjà les contraintes spécifiques en termes d'énergie électrique liées à l'une ou l'autre des utilisations, stimulation ou choc, est encore accrue par le développement des dispositifs multisite et des capteurs intracardiaques, tels que les capteurs d'accélération endocavitaire (EA).

On comprend l'avantage que peut représenter une fiche unique, soumise à un standard unique, portant une pluralité de contacts électriques permettant d'assurer simultanément l'établissement des connexions aux diverses bornes du générateur pour tous les niveaux d'énergie, qu'il s'agisse du recueil de signaux de dépolarisation, de l'application d'impulsions de stimulation ou de l'envoi d'une énergie de choc de défibrillation ou encore de la transmission de signaux recueillis par un capteur. Dans ce cadre, il a été proposé une fiche de connexion unique du type "multipolaire" et "isodiamètre" (c'est-à-dire de forme cylindrique uniforme, sans reliefs), destinée à être insérée dans une cavité cylindrique homologue de la tête de connecteur du générateur, en conformité avec la norme IS-4/DF-4 (ISO 27186-2010).

Le EP 1 641 084 A1 (ELA Médical) décrit un exemple d'une telle fiche de connexion, à quatre pôles. Plus précisément, cette fiche de connexion connue est du type isodiamètre, présentant à une de ses extrémités une broche de contact électrique axiale et, sur le corps même de la fiche, trois zones de contact électrique annulaires réalisées par des bagues cylindriques conductrices. Les zones de contact électrique annulaires sont séparées alternativement par des zones cylindriques isolantes intercalaires afin d'isoler électriquement les zones de contact électrique les unes des autres.

On peut ainsi, en enfichant d'un seul geste la fiche de connexion dans la cavité de la tête de connecteur du générateur, réaliser simultanément toutes les liaisons électriques nécessaires entre le générateur et les pôles de la sonde.

La réalisation d'une telle fiche de connexion soulève beaucoup de problèmes de fabrication. De plus, la contrainte d'un diamètre extérieur faible (3,2 mm selon ISO 27186) limite les possibilités de conception, de sorte que l'impact du respect de tolérances aussi serrées dans un cadre de production industrielle peut être considérable en termes de temps et de coût.

Une difficulté supplémentaire se présente dans le cas des sondes de type "configuration coaxiale", c'est-à-dire avec un conducteur central mobile logé dans une lumière axiale du corps de sonde, les autres conducteurs s'étendant en périphérie du corps de sonde. La broche axiale est reliée au conducteur central, avec un degré de liberté en rotation de l'ensemble broche-conducteur par rapport au corps extérieur de la fiche et du corps de sonde.

Ces sondes sont notamment utilisées pour permettre l'entraînement depuis l'extrémité proximale (là où se trouve la fiche) d'un mécanisme de déploiement d'une vis d'ancrage à l'extrémité distale, par une manipulation dite *pin-driven,* où le chirurgien maintient d'une main l'extrémité proximale du corps de sonde avec sa fiche de connexion, et fait tourner de l'autre main la broche (*pin*) dépassant de cette fiche. Le couple appliqué à la broche est transmis via le conducteur interne jusqu'au mécanisme de déploiement et d'entraînement de la vis d'ancrage, ce qui permet de contrôler la pénétration de cette dernière dans la paroi cardiaque. Une telle structure de sonde *pin-driven* est par exemple décrite dans les US 7 241 180 B1 et US 2010/0211144 A1.

La présence d'un élément mobile dans la fiche entraîne des difficultés supplémentaires de conception, du fait :
- du nécessaire respect des tolérances dimensionnelles prescrites par la norme, en dépit du jeu fonctionnel minimal nécessaire à une libre rotation de la broche axiale, et
- des impératifs d'étanchéité entre l'environnement extérieur et les régions internes de la fiche et du corps de sonde (notamment de la lumière centrale logeant le conducteur axial mobile), de façon à éviter toute pénétration de fluides corporels entre les pièces mobiles et dans les régions internes de la fiche et du corps de sonde.

Ces impératifs sont particulièrement critiques dans le cas d'une sonde portant des électrodes de défibrillation, compte tenu des énergies électriques importantes et des tensions élevées transitant dans la fiche lors de l'application du choc de défibrillation.

La structure d'une telle fiche et la manière dont la broche axiale mobile est montée sont donc des aspects particulièrement critiques, tant du point de vue de la conception de la fiche que de la mise en oeuvre du procédé de fabrication.

Le US 2010/0211144 A1 précité propose de réaliser un ensemble de montage de la broche comportant deux éléments concentriques, avec une pièce en forme d'anneau montée autour du fût de la broche (cette pièce pouvant servir de palier, si la broche est mobile à rotation) et d'autre part une collerette circonférentielle entourant l'anneau à sa périphérie et jouant un rôle de coupleur avec le corps de fiche, où cette collerette est encapsulée ou vissée lors de la fabrication.

Cette configuration permet de ménager si besoin un degré de liberté en rotation entre broche et corps de fiche. Elle conduit toutefois à une fabrication relativement complexe et coûteuse, imposant la réalisation de pièces avec des tolérances très strictes pour pouvoir respecter les contraintes dimensionnelles imposées.

Surtout, cette configuration ne procure pas de solution simple et satisfaisante aux deux séries d'exigences évoquées plus haut, à savoir :
- des tolérances très étroites malgré la présence de pièces mobiles, la broche devant pouvoir être manipulée sans jeu axial ni radial par rapport au corps de sonde, et
- l'étanchéité absolue des régions internes de la fiche et du corps de sonde par rapport à l'environnement extérieur.

Le but de l'invention est d'apporter une solution à ces différents problèmes, grâce à une configuration de fiche simplifiée, réalisable par un procédé de fabrication simple et donc peu coûteux, tout en respectant les contraintes très strictes de tolérances dimensionnelles et d'étanchéité. On verra en particulier que la mise en oeuvre industrielle de la fiche selon l'invention est facilitée par l'utilisation de pièces de formes très simples (donc faciles et peu coûteuses à usiner) et par la réduction du nombre de ces pièces à assembler.

On verra également que la configuration particulière de la fiche de l'invention assure une très bonne protection de la liaison entre la broche centrale et le conducteur intérieur auquel celle-ci est soudée, à l'égard des sollicitations diverses et des risques de rupture de cette liaison par fatigue des matériaux à l'interface broche-conducteur.

Par ailleurs, bien que l'invention soit particulièrement avantageuse dans le cadre d'une sonde de type *pin-driven* avec broche axiale mobile, on notera qu'elle peut être appliquée aussi bien à des sondes à broche axiale fixe, compte tenu des avantages évoqués plus haut, notamment de facilité de mise en oeuvre industrielle.

A cet effet, l'invention propose une fiche de connexion électrique pour sonde multipolaire de dispositif médical implantable actif telle que divulguée par exemple par le US 7 241 180 B1 précité, c'est-à-dire une fiche présentant une surface extérieure cylindrique portant une pluralité de contacts annulaires répartis axialement, et portant à son extrémité libre un contact axial, cette fiche comprenant : une broche axiale d'extrémité en matériau conducteur, formant à son extrémité libre ledit contact axial et comportant à son extrémité opposée une tige de montage apte à être reliée à un conducteur central interne de la sonde ; un corps de fiche en matériau isolant, comprenant un alésage central logeant le conducteur central et la tige de la broche et portant à sa surface extérieure les contacts annulaires, le corps de fiche logeant également des conducteurs de liaison aux contacts annulaires respectifs ; et des moyens de liaison de la broche au corps de fiche, formant interface de liaison entre la tige de la broche et la paroi intérieure de l'alésage du corps de fiche au voisinage de son débouché.

Les moyens de liaison de la broche au corps de fiche comprennent : une douille de maintien montée sur la tige de la broche de sorte que, axialement, la face frontale de cette douille vienne en appui contre un collet formé sur la tige et, radialement, la surface extérieure de cette douille vienne en contact direct avec la paroi intérieure de l'alésage du corps de fiche au débouché de celui-ci ; une bague de blocage, solidarisée à la tige de la broche de sorte que, axialement, la douille de maintien se trouve enserrée entre le collet de la tige et la bague de blocage, si besoin en laissant subsister un degré de liberté en rotation entre douille et broche ; et des moyens de solidarisation directe de la douille au corps de fiche à l'endroit de son contact avec la paroi intérieure de l'alésage.

De façon caractéristique de l'invention, les moyens de solidarisation directe de la douille au corps de fiche comprennent une liaison à encliquetage avec des crans coopérant avec une gorge homologue, liaison qui peut éventuellement être ensuite soudée ou collée.

La fiche peut en outre comprendre un joint d'étanchéité monté sur la tige de la broche de manière à se trouver enserré axialement entre la bague de blocage et un épaulement en vis-à-vis de l'alésage central du corps de fiche.

La douille est de préférence réalisée en un matériau polymère biocompatible.

On va maintenant décrire un exemple de réalisation de l'invention, en référence aux dessins annexés où les mêmes références numériques désignent d'une figure à l'autre des éléments identiques ou fonctionnellement semblables.
La Figure 1 est une vue éclatée en perspective d'une fiche de connexion selon l'invention, montrant les divers éléments constitutifs de celle-ci.
La Figure 2 est une vue perspective en coupe par un plan axial de cette même fiche, avec ses divers éléments constitutifs assemblés.
La Figure 3 illustre, isolément, en perspective et en coupe axiale, la broche centrale de la fiche de le Figure 2, avec sa douille de maintien et sa bague de blocage.
Les Figures 4 et 5 illustrent en perspective la réalisation des contacts annulaires et la formation de la liaison à leurs conducteurs respectifs.
La Figure 6 illustre en perspective le corps de fiche obtenu après surmoulage des contacts annulaires de la Figure 5.
La Figure 7 illustre, en perspective et en coupe par un plan axial, le corps de fiche de la Figure 6.
Les Figures 8, 9 et 10 illustrent trois étapes successives du processus d'assemblage de l'ensemble associé à la broche axiale et à son conducteur de liaison, et de mise en place de cet ensemble dans le corps de fiche surmoulé de la Figure 6.
La Figure 11 illustre l'état final de la fiche obtenue après exécution des diverses étapes de procédé des Figures 4 à 10.
Les Figures 12a et 12b illustrent une première forme de réalisation de la douille de maintien de la fiche de l'invention.
Les Figures 13a et 13b illustrent une deuxième forme de réalisation de la douille de maintien de la fiche de l'invention.
Les Figures 14a et 14b illustrent une troisième forme de réalisation de la douille de maintien de la fiche de l'invention.
La Figure 15 est une vue agrandie, en perspective et en coupe par un plan axial, de l'extrémité de la fiche incorporant une douille de maintien selon le deuxième mode de réalisation illustré Figures 13a et 13b.
La Figure 16 est une vue agrandie, en perspective mais non en coupe, de l'extrémité de fiche de la Figure 15.
La Figure 17 est vue agrandie, en perspective et en coupe par un plan axial, de l'extrémité de la fiche incorporant une douille de maintien selon le troisième mode de réalisation illustré Figures 14a et 14b.

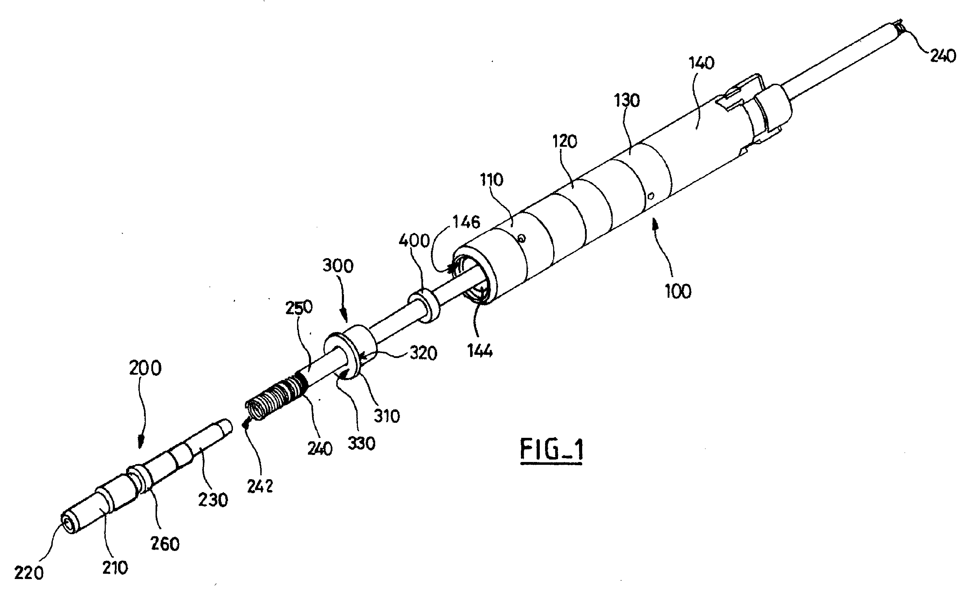

Sur les Figures 1, 2 et 11, on a représenté l'extrémité proximale d'une sonde multipolaire "isodiamètre", dans cet exemple une sonde conforme à la norme IS-4/DF-4 (ISO 27186).

Cette extrémité de sonde se présente sous la forme d'une fiche à insérer dans une tête de connecteur d'un générateur implanté, par exemple un stimulateur cardiaque, défibrillateur et/ou resynchroniseur.

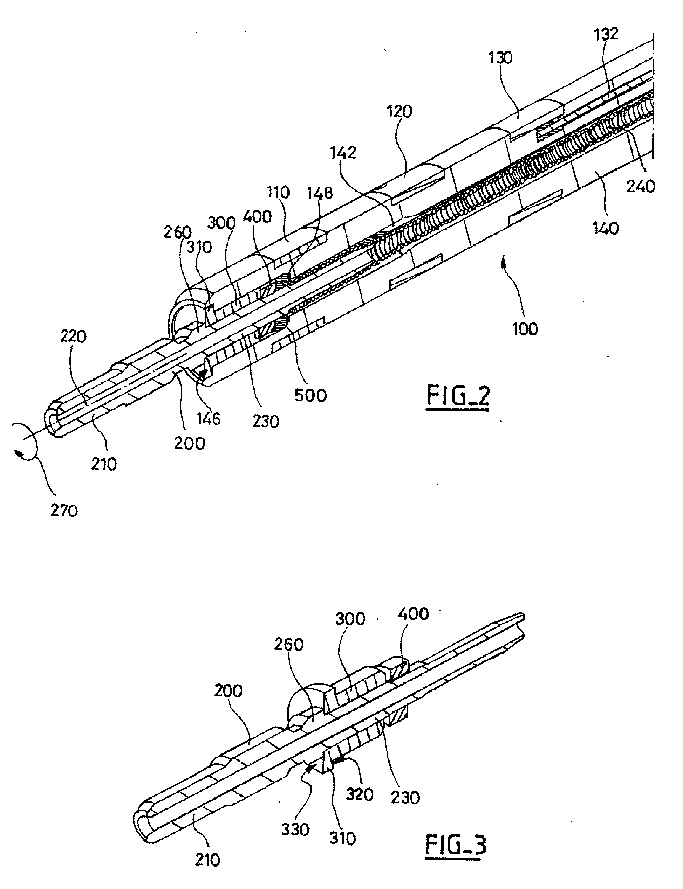

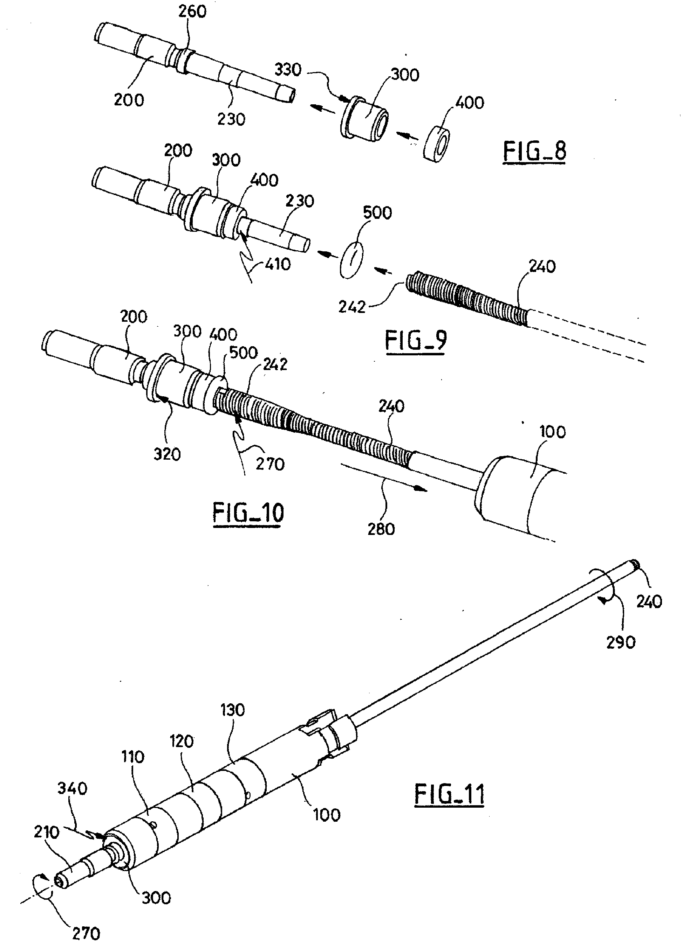

La Figure 1 est une vue éclatée de cette fiche montrant les différents éléments qui la constituent. Sur la Figure 11, la fiche est représentée sous forme assemblée, telle qu'elle se présente à l'issue du processus de fabrication, tandis que la Figure 2 montre plus en détail la structure intérieure de cette même fiche avec la configuration des différentes pièces dans leur état final, assemblées.

Cette fiche comporte un corps de fiche 100 portant une pluralité de contacts annulaires 110, 120, 130, le reste du corps de fiche étant constitué par une pièce 140 en matériau isolant. L'ensemble est de type "isodiamètre", c'est-à-dire qu'il se présente sous la forme d'un corps cylindrique lisse, dépourvu de reliefs. Ce corps de fiche 100 porte côté proximal (à gauche sur la figure) une broche axiale d'extrémité 200 en matériau conducteur, dont l'extrémité libre émergeant côté proximal de la fiche constitue un contact axial 210.

La fiche est donc une fiche quadripolaire (trois contacts annulaires et un contact axial). Cette configuration correspond par exemple à une sonde bipolaire permettant d'une part (selon la norme IS-4) le recueil des ondes de dépolarisation et l'application d'impulsions de stimulation entre l'électrode axiale 210 et l'électrode annulaire 110, et d'autre part (selon la norme DF-4) l'application d'un choc de défibrillation entre les deux électrodes annulaires 120 et 130. En variante, les électrodes 120 et/ou 130, ou d'autres électrodes annulaires additionnelles peuvent être utilisées pour une liaison à une ligne d'alimentation et/ou de commande de circuits incorporés à la sonde, à un capteur situé en bout de sonde tel qu'un capteur d'accélération endocardiaque, etc. La configuration illustrée n'est bien entendu pas limitative, et l'invention est applicable à une fiche portant un nombre quelconque de contacts annulaires.

La broche 200 présente en son centre une lumière 220 permettant l'introduction d'un mandrin pour le guidage de la sonde par le praticien au moment de l'implantation chez le patient.

A l'extrémité opposée du contact axial 210, c'est-à-dire dans la partie située à l'intérieur du corps de fiche lorsque la fiche est assemblée, la broche 200 comporte une tige de montage 230 cylindrique, dont l'extrémité située côté distal (à droite sur la figure) est destinée à être reliée à un conducteur central interne 240, à l'extrémité proximale 242 de celui-ci. Ce conducteur 240 est logé dans un alésage central 142 (Figure 2) du corps de fiche 100, et il peut le cas échéant être mobile à rotation à l'intérieur de cet alésage. Dans ce cas, le conducteur 240 est revêtu sur sa longueur d'une gaine 250 en matériau à faible coefficient de frottement, par exemple en PTFE. L'alésage central 142 débouche côté proximal en 144. La broche 200 est un élément monobloc réalisé en un matériau conducteur, par exemple un acier inoxydable MP35N. Le corps de fiche 100 est réalisé, en ce qui concerne la partie isolante 140, en un polymère biocompatible, par exemple un matériau isolant comme une polyétheréthercétone (PEEK) ou un polyuréthanne aromatique thermoplastique tel que le *Tecothane* (marque déposée), qui sont des matériaux thermoplastiques qui peuvent être aisément surmoulés avec les contacts annulaires 110, 120, 130 (qui sont par exemple réalisés en acier inoxydable MP35N), ainsi qu'avec les conducteurs de liaison à ces bagues, tels que le conducteur 132 visible Figure 2.

La liaison entre la broche 200 et le corps de fiche 100 est réalisée par un ensemble constitué d'une douille de maintien 300 enserrée entre un collet 260 formé sur la broche 200 entre l'extrémité libre 210 et la tige de montage 230, côté proximal, et une bague de blocage 400, côté distal. Ces éléments sont représentés isolément Figure 3, montés sur la broche 200. Cette configuration assure un jeu axial pratiquement nul entre la douille 300 et l'ensemble formé de la broche 200 et de la bague 400 (la bague 400 étant fixée à la broche 200 de manière à constituer un ensemble monobloc avec cette dernière), tout en ménageant si besoin un degré de liberté en rotation entre broche 200 et bague 300, pour une sonde de type *pin-driven* (cette possibilité de rotation est illustrée par la flèche 270). La douille 300 comporte côté proximal un épaulement 310 définissant, côté distal, une face 320 susceptible de venir en contact avec un épaulement homologue 146 situé au débouché 144 de la lumière interne du corps de fiche, et côté proximal une face 330 venant en appui contre le collet 260 de la broche 200.

La douille 300 est directement solidarisée au corps de fiche 100 à l'endroit de son contact avec la paroi intérieure de l'alésage interne, ce qui permet un montage très simple garantissant le respect des tolérances dimensionnelles et d'une étanchéité parfaite avec notamment l'alésage central 142 du corps de fiche 100. En ce qui concerne l'étanchéité, notamment dans le cas d'une sonde de type *pin-driven,* il est possible de prévoir également un joint torique d'étanchéité 500 (Figure 2), par exemple en silicone, comprimé entre la face distale de la bague 400 et un épaulement 148 en vis-à-vis de l'alésage central 142 du corps de fiche.

La bague 400 est réalisée en un matériau qui pourra être aisément solidarisé à la fiche de montage 230 de la broche 200, par exemple en acier inoxydable MP35N. La douille de maintien 300, quant à elle, est réalisée en un matériau qui pourra être aisément solidarisé à la partie isolante 140 du corps de fiche, par exemple en PEEK ou en *Tecothane.*

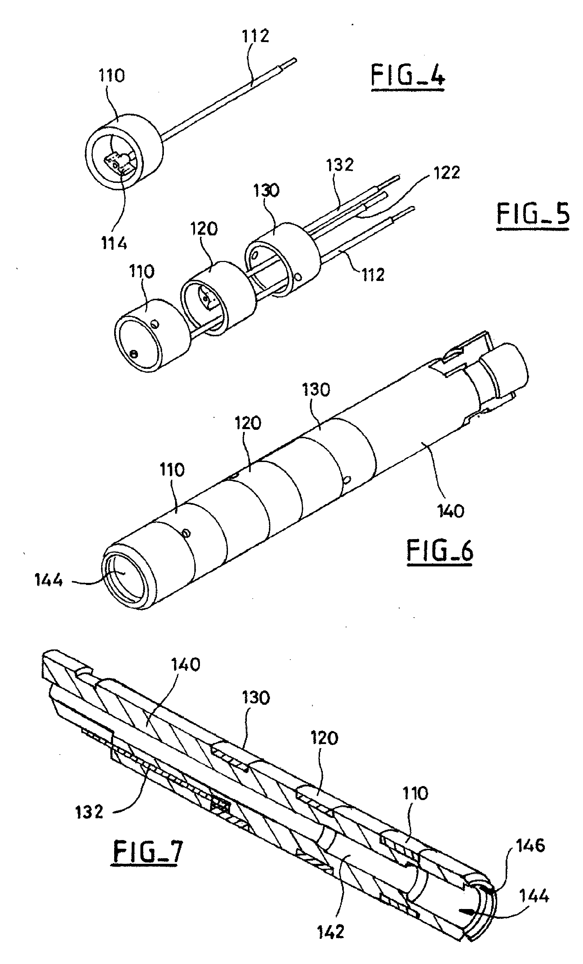

On va maintenant décrire le procédé de fabrication de la fiche que l'on vient de décrire, en référence aux Figures 4 à 11.

La première étape est la réalisation du corps de fiche 100, surmoulé avec ses contacts annulaires 110, 120, 130.

Les contacts annulaires sont préparés isolément, comme illustré Figure 4, par exemple en soudant à l'intérieur d'un contact 110 une cosse 114 sur laquelle est soudé ou serti le conducteur correspondant 112 (le contact 110 et la cosse 114 pouvant être, en variante, deux parties d'une même pièce monobloc).

Comme illustré Figure 5, les divers contacts annulaires 110, 120, 130 munis de leurs conducteurs respectifs 112, 122, 132 sont ensuite positionnés les uns par rapport aux autres selon la configuration qu'ils devront avoir sur le corps de fiche dans son état final.

La partie isolante 140 du corps de fiche, en matériau polymère biocompatible, est surmoulée avec les contacts 110, 120, 130 comme illustré Figure 6, en ménageant une lumière centrale 142.

Le sous-ensemble ainsi obtenu est illustré Figure 6, et Figure 7 en coupe. On notera en particulier qu'au débouché 144 de l'alésage central 142 on ne trouve aucune pièce ou insert surmoulé, seulement le débouché 144 avec l'épaulement 146 destiné à recevoir ultérieurement la douille de maintien. En d'autres termes, la pièce obtenue à ce stade du processus est une pièce intégralement réalisée en matériau isolant, à l'exception des contacts annulaires et de leurs conducteurs de liaison.

Séparément, la broche et les pièces servant à constituer son interface de liaison sont assemblées, comme illustré Figures 8, 9 et 10.

La première étape (Figure 8) consiste à enfiler la douille de maintien 300 sur la tige de montage 230 de la broche 200, jusqu'à ce que la face 330 de cette douille vienne en butée contre le collet 260. La bague de blocage 400 est ensuite enfilée également sur la tige de montage 230 de manière à plaquer la bague 300 contre le collet 260, puis cette bague est solidarisée à la broche 200 dans cette position par exemple par soudage laser (comme illustré en 410, Figure 9), collage, vissage, sertissage ou autre moyen de solidarisation approprié.

L'étape suivante, illustrée Figure 9, consiste, après avoir placé le joint optionnel 500 autour de la tige de montage 230 et contre la bague 400, à souder l'extrémité 242 du conducteur axial 240 sur l'extrémité de la tige de montage 230, par exemple par soudage laser (comme illustré en 270, Figure 10). Le sous-ensemble ainsi obtenu est illustré Figure 10 dans son état final.

L'étape suivante, illustrée Figure 10, consiste à introduire (flèche 280) ce sous-ensemble dans la lumière intérieure du corps de fiche 100 à partir de l'extrémité proximale de ce dernier, jusqu'à ce que la face 320 de la douille de maintien 300 côté proximal vienne en butée contre le matériau plastique du corps 140.

Ces deux éléments sont alors solidarisés entre eux, par exemple par soudage laser (comme illustré en 340, Figure 11), collage, soudage aux ultrasons, soudage chimique ou tout autre procédé approprié.

Dans une variante de ce procédé, le conducteur axial 240 est préparé en même temps que le corps de fiche 100, et constitue avec lui un ensemble propre. Le conducteur axial 240 de ce dernier est alors monté sur un autre ensemble, qui est celui illustré Figure 8, comprenant la broche 200, la douille de maintien 300 et la bague de blocage 400 assemblés entre eux, avec insertion éventuelle du joint d'étanchéité 500 sur la tige de la broche avant le soudage. Le conducteur axial 240 est soudé sur la tige de montage 230, et la configuration résultante est alors identique à celle décrite plus haut et illustrée Figure 10.

La Figure 11 illustre l'état final de la fiche obtenue après exécution des diverses étapes de procédé des Figures 4 à 10.

On soulignera que la solidarisation des deux sous-ensembles (à savoir le corps de fiche résultant des étapes des Figures 5 à 7, et la broche pourvue des différents éléments associés résultant des étapes des Figures 8 et 9) est réalisée directement, sans intermédiaire.

On notera en particulier que, comme la douille 300 et le polymère 140 du corps de fiche sont réalisés dans des matériaux identiques ou comparables (deux polymères biocompatibles), il est aisé de réaliser une telle solidarisation avec toutes les garanties de respect des tolérances dimensionnelles et d'étanchéité, sans mise en oeuvre de techniques de fabrication particulièrement complexes.

Enfin et surtout, on notera que ces exigences sont respectées même dans une configuration de type *pin-driven* offrant un degré de liberté en rotation entre la broche d'extrémité 210 et le corps de fiche 100, c'est-à-dire dans laquelle une rotation 270 imprimée à la broche axiale 210 est transmise (comme illustré en 290 sur la Figure 11) sur toute la longueur du conducteur central 240.

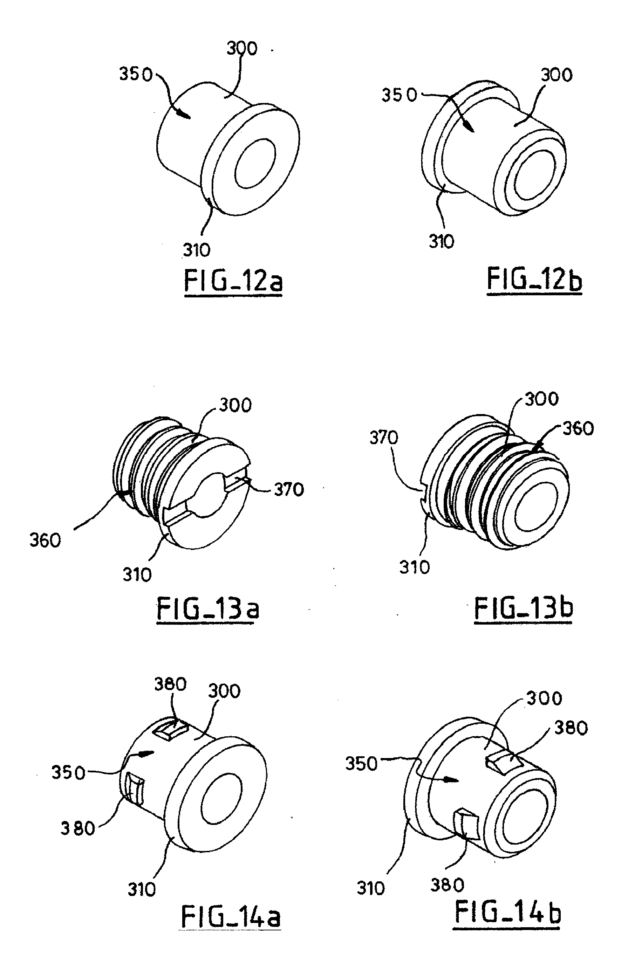

Sur les Figures 12 à 17 on a illustré différentes formes de réalisation de la douille de maintien 300.

Les Figures 12a et 12b correspondent à un premier mode de réalisation, qui est celui illustré sur les Figures 1 à 11 que l'on vient de décrire, avec une surface extérieure 350 lisse introduite dans un évidement de forme conjuguée situé au débouché du corps de fiche, où cette bague est collée ou soudée.

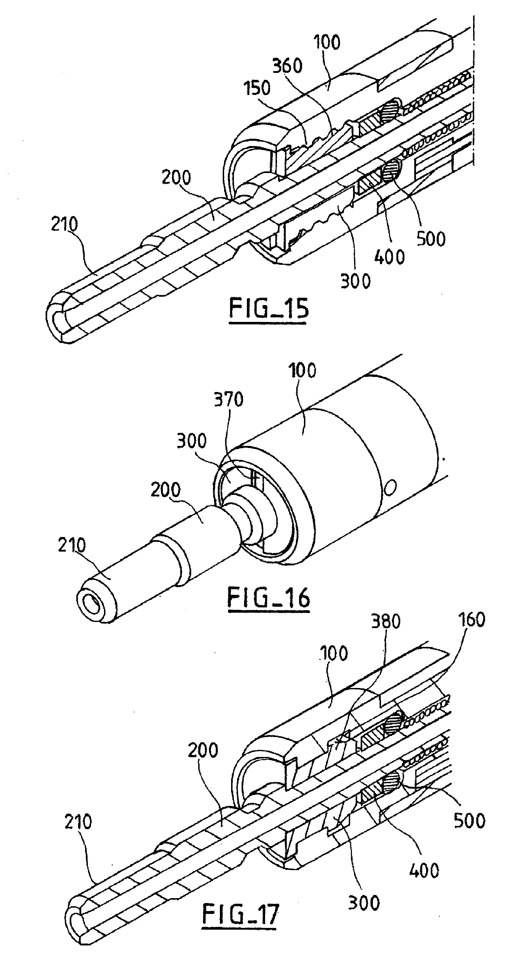

Un deuxième mode de réalisation est illustré Figures 13a et 13b, ainsi que sous forme assemblée sur les Figures 15 et 16. Dans ce mode de réalisation, la surface extérieure de la douille 300 est pourvue d'un filetage 360 coopérant avec un taraudage homologue 150 formé dans le corps de fiche 100 (Figure 15). Sur la face frontale apparente, il est possible de prévoir une empreinte 370 (voir également la Figure 16) pour un outil approprié permettant de réaliser le vissage de la douille 300 dans le corps de fiche. Pour renforcer la solidarisation de ces deux éléments, il peut être prévu en outre de les coller ou de les souder ensemble.

Dans un troisième mode de réalisation, caractéristique de l'invention, illustré Figures 14a, 14b et 17, la surface extérieure 350 de la douille 300 est pourvue de crans 380 coopérant avec une gorge homologue 160 (Figure 17) formée dans le corps de fiche. Ceci permet une mise en place de la douille 300 par encliquetage direct dans le corps de fiche, avec si besoin collage ou soudage de ces deux éléments pour renforcer la liaison entre eux.

## Revendications

1. Une fiche de connexion électrique pour sonde multipolaire de dispositif médical implantable actif, cette fiche présentant une surface extérieure cylindrique portant une pluralité de contacts annulaires (110, 120, 130) répartis axialement, et portant à son extrémité libre un contact axial (210), cette fiche comprenant :
- une broche axiale d'extrémité (200) en matériau conducteur, formant à son extrémité libre ledit contact axial (210) et comportant à son extrémité opposée une tige de montage (230) apte à être reliée à un conducteur central interne (240) de la sonde ;
- un corps de fiche (140) en matériau isolant, comprenant un alésage central (142) logeant le conducteur central et la tige de la broche et portant à sa surface extérieure les contacts annulaires (110, 120, 130), le corps de fiche logeant également des conducteurs de liaison (112, 122, 132) aux contacts annulaires respectifs ; et
- des moyens de liaison de la broche au corps de fiche, formant interface de liaison entre la tige de la broche et la paroi intérieure de l'alésage du corps de fiche au voisinage de son débouché,
dans laquelle les moyens de liaison de la broche au corps de fiche comprennent :
- une douille de maintien (300) montée sur la tige (230) de la broche de sorte que :
· axialement, la face frontale (310) de cette douille vienne en appui contre un collet (260) formé sur la tige, et
· radialement, la surface extérieure de cette douille vienne en contact direct avec la paroi intérieure de l'alésage du corps de fiche (140) au débouché de celui-ci ;
- une bague de blocage (400), solidarisée à la tige (230) de la broche de sorte que, axialement, la douille de maintien (300) se trouve enserrée entre le collet de la tige et la bague de blocage, si besoin en laissant subsister un degré de liberté en rotation entre douille et broche ; et
- des moyens de solidarisation directe de la douille (300) au corps de fiche (140) à l'endroit de son contact avec la paroi intérieure de l'alésage,
**caractérisée en ce que** les moyens de solidarisation directe de la douille au corps de fiche comprennent une liaison à encliquetage avec des crans (380) coopérant avec une gorge (160) homologue.

2. La fiche de connexion de la revendication 1, comprenant en outre un joint d'étanchéité (500) monté sur la tige de la broche de manière à se trouver enserré axialement entre la bague de blocage (400) et un épaulement en vis-à-vis (148) de l'alésage central (142) du corps de fiche.

3. La fiche de connexion de la revendication 1, dans laquelle la douille (300) est réalisée en un matériau polymère biocompatible.

4. La fiche de connexion de la revendication 1, dans laquelle ladite liaison à encliquetage est en outre soudée ou collée.

## Patentansprüche

1. Elektrischer Anschlussstecker für eine mehrpolige Sonde einer aktiven implantierbaren medizinischen Vorrichtung, wobei dieser Stecker eine zylindrische Außenfläche aufweist, die eine Vielzahl von axial verteilten Ringkontakten (110, 120, 130) trägt, und an seinem freien Ende einen axialen Kontakt (210) trägt, wobei dieser Stecker enthält:
- einen axialen Endstift (200) aus leitendem Material, der an seinem freien Ende den axialen Kontakt (210) bildet und an seinem gegenüberliegenden Ende eine Montagestange (230) aufweist, die mit einem inneren zentralen Leiter (240) der Sonde verbunden werden kann;
- einen Steckerkörper (140) aus Isoliermaterial, der eine zentrale Bohrung (142) enthält, die den zentralen Leiter und die Stange des Stifts aufnimmt, und an seiner Außenfläche die Ringkontakte (110, 120, 130) trägt, wobei der Steckerkörper ebenfalls Leiter (112, 122, 132) zur Verbindung mit den jeweiligen Ringkontakten aufnimmt; und
- Einrichtungen zur Verbindung des Stifts mit dem Steckerkörper, die eine Verbindungsschnittstelle zwischen der Stange des Stifts und der Innenwand der Bohrung des Steckerkörpers in der Nähe ihrer Ausmündung formen,
wobei die Einrichtungen zur Verbindung des Stifts mit dem Steckerkörper enthalten:
- eine Haltehülse (300), die so auf die Stange (230) des Stifts montiert ist, dass:
· axial die Stirnseite (310) dieser Hülse gegen einen auf der Stange geformten Kragen (260) in Anlage kommt, und
· radial die Außenfläche dieser Hülse in direkten Kontakt mit der Innenwand der Bohrung des Steckerkörpers (140) an deren Ausmündung kommt;
- einen Arretierring (400), der fest mit der Stange (230) des Stifts verbunden ist, so dass die Haltehülse (300) sich axial zwischen dem Kragen der Stange und dem Arretierring eingeklemmt befindet, notfalls, indem ein Drehfreiheitsgrad zwischen Hülse und Stift gelassen wird; und
- Einrichtungen zur direkten festen Verbindung der Hülse (300) mit dem Steckerkörper (140) an der Stelle ihres Kontakts mit der Innenwand der Bohrung,
**dadurch gekennzeichnet, dass** die Einrichtungen zur direkten festen Verbindung der Hülse mit dem Steckerkörper eine Einrastverbindung mit Rasten (380) enthalten, die mit einer entsprechenden Rille (160) zusammenwirken.

2. Anschlussstecker nach Anspruch 1, der außerdem eine Dichtung (500) enthält, die so auf die Stange des Stifts montiert ist, dass sie axial zwischen dem Arretierring (400) und einer gegenüberliegenden Schulter (148) der zentralen Bohrung (142) des Steckerkörpers eingeklemmt wird.

3. Anschlussstecker nach Anspruch 1, wobei die Hülse (300) aus einem biokompatiblen Polymermaterial hergestellt ist.

4. Anschlussstecker nach Anspruch 1, wobei die Einrastverbindung außerdem geschweißt oder geklebt ist.

## Claims

1. An electric connection plug for a multipolar lead of active implantable medical device, said plug having a cylindrical external surface carrying a plurality of axially-distributed annular contacts (110, 120, 130), and carrying at its free end an axial contact (210), said plug comprising:
- an end axial pin (200) made of a conductive material, forming at its free end said axial contact (210) and comprising at its opposite end a mounting rod (230) adapted to be connected to an internal central conductor (240) of the lead ;
- a plug body (140) made of an insulating material, comprising a central hole (142) accommodating the central conductor and the pin's rod and carrying at its external surface the annular contacts (110, 120, 130), the plug body also accommodating conductors (112, 122, 132) for coupling to the respective annular contacts ; and
- means for coupling the pin to the plug body, forming a coupling interface between the pin's rod and the inner wall of the plug body's hole, near the opening thereof, wherein the means for coupling the pin to the plug body comprise:
- a holding sleeve (300) mounted on the rod (230) of the pin, so that:
· axially, the front face (310) of this sleeve comes in rest against a collar (260) formed on the rod, and
· radially, the external surface of this sleeve comes into contact with the inner wall of the hole of the plug body (140), at the opening thereof ;
- a locking ring (400), fastened to the pin's rod (230) so that, axially, the holding sleeve (300) is enclosed between the rod's collar and the locking ring, with, if need be, a rotational degree of freedom being left between the sleeve and the pin ; and
- means for direct fastening of the sleeve (300) to the plug body (140) at its contact with the inner wall of the hole,
**characterized in that** the means for direct fastening of the sleeve to the plug body comprise a catch-coupling with catches (380) cooperating with a counter-part groove (160).

2. The connection plug of claim 1, further comprising a sealing gasket (500) mounted on the pin's rod so as to be axially enclosed between the locking ring (400) and an opposite shoulder (148) of the central hole (142) of the plug body.

3. The connection plug of claim 1, wherein the sleeve (300) is made of a biocompatible polymer material.

4. The connection plug of claim 1, wherein said catch-coupling is further welded or bonded.
